# EUROPEAN PATENT APPLICATION

(11) **EP 2 631 300 A1**
(43) Date of publication of application: **28.08.2013**
(21) Application number: 12001210.9
(22) Date of filing: 23.02.2012
(51) Int. Cl.: C12Q 1/68

(54) **Detection of leishmania**

(71) Applicant: Biosure R & T Cell Co., Athens (GR); Ikonomopoulos, John, 152 34 Halandri (GR)
(72) Inventor: Gazouli, Maria, 116-33 Athens (GR); Liandris, Emmanouil, 113-61 Athens (GR); Ikonomopoulos, John, 152-34 Halandri, Athens (GR); Tachtsidis, Ilias, London, SE25 5HJ (GB); Goutas, Nicholaos, 152-37 Filothei, Athens (GR); Vlachodimitropoulos, Dimitrios, 151-22 Marousi, Athens (GR); Taka, Stiliani, 121-31 Peristeri (GR); Andreadou, Margarita, 184-51 Nikaia (GR)
(74) Representative: Malamis, Alkisti-Irene

(57) **Abstract**

The invention describes the detection of *Leishmania* by use of *Leishmania*-specific oligonucleotides sequences whose physico-chemical characteristics will be adequately variable to allow their further conjugation to gold nanoparticles (AuNPs) in a way that will secure optimum performance (for example in systems based on AuNPs).

## Description

### Field of the invention

The general technical field of the invention relates to *Leishmania* detection.

### Background to the invention

*Leishmania* parasites are the etiological agents of leishmaniasis. The parasites are transmitted to mammals by the bite of phlebotomine sand flies and occasionally by congenital transmission or iatrogenically by sharing of needles and blood transfusion (Murray et al., 2005). It is manifested in a number of forms in humans, i.e. cutaneous, mucocutaneous and visceral. The latter is considered a serious and often fatal health threat, especially in endemic areas. Dogs (*Canis familiaris)* are the principal reservoir hosts of *Leishmania infantum,* causing zoonotic visceral leishmaniosis in the Mediterranean basin (Ashford et al., 1998).

In terms of global burden of disease, leishmaniasis is the third most important vector-borne disease, and it is estimated that worldwide there are an annual 1.5 to 2 million cases, with up to 350 million people at risk of infection and disease. Surveillance data indicate that the global number of cases has increased in recent decades, and several important epidemics have been reported (e.g., Sudan and Afghanistan). Such increases can be explained, in part, by improved diagnosis and case notification but are also due to other factors such as inadequate vector or reservoir control; increased detection of disease associated with opportunistic infections (e.g., human immunodeficiency virus [HIV]/AIDS), urbanization, and deforestation; the emergence of antileishmanial drug resistance; economic hardship; armed conflict; and tourism.

The reason why leishmaniasis is such a diagnostic challenge is because of the wide spectrum of clinical manifestations that they may present. Very often infected humans and dogs do not show clinical signs while symptoms when present, maybe similar to those of several other diseases (Dantas-Torres et al., 2006; Mohebali et al., 2005; Moreno and Alvar, 2002; Moshfe et al., 2009). Moreover, the periodical presence of the pathogen in blood and the development of animals-carriers complicate the diagnosis of the disease.

Today, diagnosis is based mainly on the detection of specific antibodies via immunofluorescence or the detection of the parasite by microscopy, culture or molecular techniques such as Polymerase Chain Reaction (PCR). Each of these methods has certain advantages and limitations but in general, those methods with high specificity and low minimum detection limit are usually complex and expensive. In most cases the reliable application of these diagnostic methods requires highly trained personnel and very often, dedicated equipment that can be of considerable cost. Therefore, the development of a new diagnostic assay that would be easily applicable even by non-specialized personnel, and would allow specific and sensitive detection and identification of *Leishmania* directly from clinical samples without the need of high cost dedicated equipment, would definitely improve diagnostic investigation of parasite infections.

### Summary of the invention

The invention concerns *Leishmania*-specific oligonucleotides sequences whose physicochemical characteristics will be adequately variable to allow their further conjugation to gold nanoparticles (AuNPs) in a way that will secure optimum performance (for example in systems based on AuNPs).

To this goal we have constructed a set of oligonucleotide probes that bind selectively to highly conserved region of the *Leishmania* genome and can be conjugated AuNPs, aiming to the detection of *Leishmania* DNA without molecular amplification.

The use of AuNPs-universal-probes allows rapid and direct detection collectively of the main *Leishmania* pathogens [*Leishmania infantum, Leishmania donovani, Leishmania major, Leishmania tropica*] in clinical samples, in a way that is highly specific, very easy to perform, and requires minimum infrastructure and expertise.

Accordingly the invention provides a method of determining the presence of *Leishmania* polynucleotides in a sample comprising contacting the sample with one or more oligonucleotide probes and determining whether the oligonucleotides bind to *Leishmania* polynucleotides in the sample, wherein:
- at least one of the oligonucleotide probe is attached to AuNPs, and oligonucleotide probe is chosen from any of the oligonucleotides shown in table 2 or a sequence which is at least 90% homologous to any of the sequences shown in table 2.

### Detailed description of the invention

The oligonucleotides aimed to be used for conjugation with 20 nm AuNPs were designed to be 20 bases long whereas a thiol group and an (A)₁₀ tail were added at the 5' end of the oligonucleotides in order to be used for its conjugation with AuNPs.

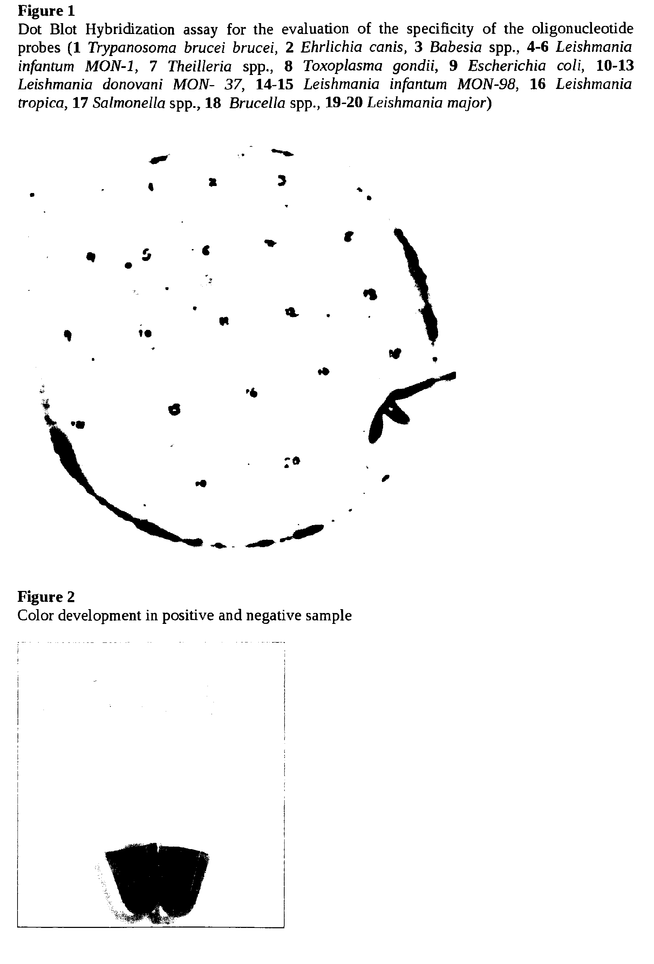

The *Leishmania* target site was selected from kinetoplastid minicircle DNA of *Leishmania* spp., using the Primer Premier software. At this stage the work involved selection of only those oligonucleotide candidates that did not produce secondary structures and retained minimum annealing tendency to each other, so that they could be used all together. The BLAST-N v. 2.2.6 tool (National Center for Biotechnology Information, www.ncbi.nlm.nih.gov) was used to confirm that none of the selected oligonucleotides recognized any registered DNA sequence other than those that were target-based. In total, a set of 5 oligonucleotides was finally constructed in this way. The specificity of the designed probes was further evaluated by Dot Blot Hybridization assays (Figure 1) with a panel of DNA samples extracted from various *Leishmania* species and other pathogens commonly found in clinical samples (Table 1). The present invention discloses the construction of highly specific oligonucleotide probes that can be used for identification of *Leishmania* and at the same time for conjugation with AuNPs.

We list the oligonucleotides that have proved to be more reliable for detection of *Leishmania* spp. (Table 2).

Colloidal gold nanoparticles (AuNPs) range in size from 3-100 nm, they are stable, uniform and monodispersed particles that can be easily produced by various methods among which citrate reduction of chloroauric acid. AuNPs exhibit strong size-dependent optical resonance, tunable for emission in the near-infrared (NIR) part of the spectrum, which is generally known as surface plasmon resonance (SPR). This resonance is pronounced in noble metal NPs because of the collective oscillations of conduction band electrons. The SPR is especially interesting with reference to Au (but also silver (Ag)) NPs. Under photoactivation, the plasmon couples with the excitation light and produces huge enhancement of the electromagnetic (EM) field in metal NPs. Interactions between the incident light and the oscillating electric fields result in the scattering and absorption of light. The enormous enhancement of the local EM field due to the coupling of the oscillating electric fields in metal NPs with the incident light make them attractive for applications from optical devices to bioanalyses and bioimaging and can be exploited to generate a color change (visual detection) or heat (construction of biosensors).

AuNPs are negatively charged and are very sensitive to changes of the solution dielectric constant (Niemeyer et al., 2004). This sensitivity is significant in terms of their potential application in diagnostics, since for typical citrate stabilized particles the addition of NaCl neutralizes the surface charge and can cause a decrease in inter-particle distance. This can be directed to selective deposition of the AuNPs that will be eventually manifested by a change of color detectable by visual observation (Niemeyer et al., 2004). The same result can be achieved by linkage of two or more functionalized (modified chemically for conjugation) AuNPs to a target molecule of DNA, which results to aggregation because it decreases the distance between the particles.

Depending on the application that is being developed the properties of AuNPs have been incorporated to the construction of different detection mechanisms i.e. visual observation of color change or measurement of optical absorption. In all cases, the basic idea is that in solution monodisperse AuNPs appear pink and exhibit a relatively narrow surface plasmon absorption band centered at around 520 nm in the UV-visible spectrum. In contrast a solution containing aggregated AuNPs appears purple in color corresponding to a characteristic pink shift in the surface plasmon resonance of the particles from 520 nm to 574 nm. Colloidal gold offer some unique features such as, they do not undergo any photodecomposition, which is a common problem encountered while using fluorescent dyes. Secondly, they are not toxic, in sharp contrast to potential toxicity of semiconductor QDs. Thirdly, they are reasonably stable and can be stored in dry state as well. Lastly, their ability to shift the Surface Plasmon Resonance (SPR), in a controlled fashion, to the spectral region best suited for optical bioimaging and biosensing applications. Therefore the use of these nanoparticles as biodetectors relies either on the shift of absorption that can be accurately measured or be detected visually by the resulting change of color.

Their integration to diagnosis of Leishmania could greatly improve the detection limit of the technique without the need of amplification of the parasite DNA.

Nucleotide probes conjugated with gold nanoparticles (AuNP-probes) were first used for this purpose a few years ago for the detection of M. tuberculosis DNA (Baptista et al., 2006). AuNP-probes are already integrated in research and routine diagnostic applications and have shown great potential (Chen et al., 2008).

AuNPs seem to be very promising as highly sensitive biosensors, since they already constitute the basis of a very innovative diagnostic approach. The ability to detect very small amounts of the AuNPs can decrease dramatically the lower detection limit of the diagnostic assays in which they are incorporated, allowing direct, even in-field detection of microbial nucleotide sequences, without the need for amplification. AuNPs are very promising as they could be used for the simultaneous quantitative detection of different targets. A great number of samples can be processed at the same time while the use of portable devices could permit the in-field detection of the AuNPs.

It becomes evident that a method that will combine the advantages of the diagnostic tests mentioned above will greatly improve diagnostic investigation of *Leishmania* infections.

The problem arises in that biomolecules designated to be used with AuNPs should be designed in a way that secure specificity for what they will detect and will render them at the same time, suitable for AuNPs -conjugation.

Aim of the study that led to the present invention was the development of five oligonucleotides that could
- detect the major pathogenic *Leishmania* species in a sensitive and specific way and
- fulfill the requirements for the stable conjugation of these molecules to AuNPs.

In one embodiment of the method it is carried out to diagnose infection of an individual suspected to have a leishmanial infection, wherein the individual is preferably human. Homologues of oligonucleotide sequences are referred to herein. Such homologues typically have at least 70% homology, preferably at least 80, 90%, 95%, 97% or 99% homology, for example over a region of at least 15, 20, 30 or more contiguous nucleotides, or even across the entire length of the original sequence. The homology may be calculated on the basis of nucleotide identity (sometimes referred to as "hard homology").

For example the UWGCG Package provides the BESTFIT program which can be used to calculate homology (for example used on its default settings) (Devereux et al (1984) Nucleic Acids Research 12, p387-395). The PILEUP and BLAST algorithms can be used to calculate homology or line up sequences (such as identifying equivalent or corresponding sequences (typically on their default settings), for example as described in Altschul S. F. (1993) J Mol Evol 36:290-300; Altschul, S, F et al (1990) J Mol Biol 215:403-10.

Software for performing BLAST analyses is publicly available through the National Center for Biotechnology Information (http://www.ncbi.nlm.nih.gov/). This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul *et al,* supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl. Acad. Sci. USA 90: 5873-5787. One measure of similarity provided by the BLAST algorithm is the smallest sum probability (P(N)), which provides an indication of the probability by which a match between two oligonucleotide sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

The homologous sequence typically differs by 1, 2, less than 5, less than 10 or less than 15 mutations (each of which may be a substitution, deletion or insertion of a nucleotide). These mutations may be measured across any of the regions mentioned above in relation to calculating homology.

All publications which are mentioned above and below are incorporated herein by reference. The following Examples illustrate the invention.

### Example 1

### Method Description

AuNPs purchased from Nanopartz were conjugated with five thiolated oligonucleotide specific for the *Leishmania* spp. using a previously described protocol (Hill and Mirkin 2006). The oligonucleotides sequence is listed above (Table 2).

DNA isolation from *Leishmania* cultures was performed using commercial kits.

Specifically, 10µl DNA were diluted in 50 µl of 10mM PBS (pH 5.0). After denaturation at 95 °C for 5 min, the solution was cooled to 65 °C and 10 µl of each AuNP-probe was added followed by 20 µl of 0.01N HCl, after 5 min of incubation at room temperature. Blank preparations were made in exactly the same way using an equivalent volume of 10mMPBS instead of DNA. The solutions were kept for 10-20 min at room temperature until color was developed and then they were photographed. The color could be detected visually and it was confirmed with an absorption spectrum.

In positive samples the color of the solution remained pink, while in the absence of target DNA the solution turned purple (Figure 2).

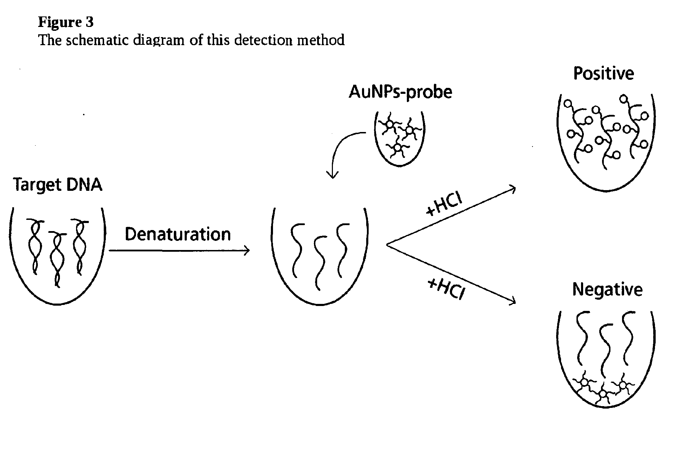

The schematic diagram of this detection method is shown in Figure 3.

Our results indicate the AuNPs-probe method may offer fast and reliable *Leishmania* DNA detection, without amplification of DNA. Furthermore, the AuNPs -probe assay reaction is performed in a single tube, which reduces carryover contamination. The method allows simple visual detection of the result and it does not necessarily require dedicated high-cost equipment.

### References

Ashford, D.A., David, J.R., Freire, M., David, R., Sherlock, I., Eulalio, M.C., Sampaio, D.P., Badaro, R., 1998. Studies on control of visceral leishmaniosis impact of dog control on canine and human visceral leishmaniasis in Jacobina, Bahia, Brazil. Am. J. Trop. Med. Hyg. 59,53-57.
Baptista PV, Koziol-Montewka M, Paluch-Oles J, Doria G, Franco R. Gold-nanoparticle-probe-based assay for rapid and direct detection of Mycobacterium tuberculosis DNA in clinical samples. Clin Chem 2006; 52: 1433-4
Chen H, Wu V, Chuang Y, Lin C. Using oligonucleotide-functionalized Au nanoparticles to rapidly detect foodborne pathogens on a piezoelectric biosensor. J Microbiol Meth 2008; 73: 7-17
Dantas-Torres, F., Brito, M.E.F., Brandão-Filho, S.P., 2006. Seroepidemiological survey on canine leishmaniasis among dogs from an urban area of Brazil. Vet. Parasitol. 40, 54-60.
Hill, D.H., Mirkin, C.A., 2006. The bio-barcode assay for the detection of protein and nucleic acid targets using DTT-induced ligand exchange. Natl. Protoc. 1, 324-336.
Mohebali, M., Hajjaran, H., Hamzavi, Y., Mobedi, I., Arshi, S., Zarei, Z., Akhoundi, B., Naeini, K.M., Avizeh, R., Fakhar, M., 2005. Epidemiological aspects of canine visceral leishmaniosis in the Islamic Republic of Iran. Vet. Parasitol. 129, 243-251.
Moreno, J., Alvar, J., 2002. Canine leishmaniasis: epidemiological risk and the experimental model. Trends Parasitol. 18, 399-405.
Moshfe, A., Mohebali, M., Edrissian, G., Zarei, Z., Akhoundi, B., Kazemi, B., Jamshidi, S., Mahmoodi, M., 2009. Canine visceral leishmaniasis: Asymptomatic infected dogs as a source of L. infantum infection. Acta Trop. 112, 101-105.
Murray, H.W., Berman, J.D., Davies, C.R., Saravia, N.G., 2005. Advances in leishmaniasis. Lancet 366, 1561-1577.
Niemeyer CM, Mirkin CA. Nanobiotechnology. Singapore: Markono print media Pte Ltd, 2004.

**Table 1**

| Strains used as controls for the evaluation of the specificity of the oligonucleotide probes | |
|---|---|
| **Pathogens** | **Origin** |
| *Leishmania infantum MON-1* | University of Crete, Faculty of Medicine |
| *Leishmania infantum MON-98* | University of Crete, Faculty of Medicine |
| *Leishmania donovani MON- 37* | University of Crete, Faculty of Medicine |
| *Leishmania tropica* | University of Crete, Faculty of Medicine |
| *Leishmania major* | University of Crete, Faculty of Medicine |
| *Trypanosoma brucei brucei* | University of Crete, Faculty of Medicine |
| *Ehrlichia canis* | Agricultural University of Athens, Dep. of Animal Science and Aquaculture |
| *Babesia* spp. | Agricultural University of Athens, Dep. of Animal Science and Aquaculture |
| *Theilleria* spp. | Agricultural University of Athens, Dep. of Animal Science and Aquaculture |
| *Toxoplasma gondii* | Agricultural University of Athens, Dep. of Animal Science and Aquaculture |
| *Escherichia coli* | Agricultural University of Athens, Dep. of Animal Science and Aquaculture |
| *Salmonella* spp. | Agricultural University of Athens, Dep. of Animal Science and Aquaculture |
| *Brucella* spp. | Agricultural University of Athens, Dep. of Animal Science and Aquaculture |

**Table 2**

| Oligonucleotide probes for AuNPs conjugation, specific for *Leishmania* detection | | | |
|---|---|---|---|
| **Name** | **Target** | **Sequence 5'-3'** | **Tm** |
| Leish Au1 | kinetoplastid minicircle DNA | Thiol-AAAAAAAAAAGTTAGCCGATGGTGGTCTTG | 63.2 |
| Leish Au2 | kinetoplastid minicircle DNA | Thiol-AAAAAAAAAAACGGGTGTCTTTGATGATGC | 63.8 |
| Leish Au3 | kinetoplastid minicircle DNA | Thiol-AAAAAAAAAATAGTCTGGTGGGATGCTTCG | 63.2 |
| Leish Au4 | kinetoplastid minicircle DNA | Thiol-AAAAAAAAAAGTGCCTTTGATGTGGGTGTT | 63.5 |
| Leish Au5 | kinetoplastid minicircle DNA | Thiol-AAAAAAAAAAGCTAGCGTTTTTGGTGTTTT | 60.9 |

## Claims

1. A method of determining the presence of *Leishmania* DNA in a sample comprising contacting the sample with one or more oligonucleotide probes and determining whether the oligonucleotides bind to *Leishmania* DNA in the sample, wherein:
- at least one of the oligonucleotide probes is attached to AuNPs, and the oligonucleotide probe is chosen from any of the oligonucleotides shown in tables 2 or a sequence which is at least 90% homologous to any of the sequences shown in tables 2.

2. A method according to claim 1 which is carried out to diagnose infection of an individual suspected to have a leishmanial infection, wherein the individual is preferably human.

3. A method according to claim 1 or 2 wherein said contacting is performed by adding the oligonucleotide probes to polynucleotides and is able to bind by Watson-Crick base pairing to polynucleotide sequence in the sample.

4. A method according to claim 3 wherein the oligonucleotide probes are chosen from any of the oligonucleotides shown in tables 2.

5. A method according to any one of claims 3 and 4 wherein the oligonucleotide probesare labeled with thiol which is bound to AuNPs.

6. A method according to any one of the preceding claims in which the DNA in the sample has not been amplified and/or the sample is from an individual who does not have any symptoms of leishmanial infection or disease.

7. A method according to any one of the preceding claims wherein all of the oligonucleotides shown in Table 2, or homologues thereof, are used.

8. A kit for carrying out the method of any one of claims 1 to 7 comprising 1, 2, 3, 4 or 5 oligonucleotides as defined in claim 1.
